# EUROPEAN PATENT APPLICATION

(11) **EP 4 046 580 A1**
(43) Date of publication of application: **24.08.2022**
(21) Application number: 20890989.5
(22) Date of filing: 28.10.2020
(51) Int. Cl.: A61B 17/122, A61B 17/128

(54) **HEMOSTATIC CLIP AND AUXILIARY SYSTEM**

(30) Priority: 18.11.2019 CN 201911128624
(71) Applicant: MicroPort Urocare (Jiaxing) Co., Ltd., Nanhu District Jiaxing Zhejiang 314006 (CN); Shi, Yihai, Shanghai 201203 (CN)
(72) Inventor: WANG, Zhen, Shanghai 201203 (CN); JIANG, Lu, Shanghai 200135 (CN); WANG, Changsheng, Shanghai 200135 (CN); LI, Penghui, Jiaxing, Zhejiang 314006 (CN); BAO, Shiyong, Jiaxing, Zhejiang 314006 (CN); HUAN, Qian, Shanghai 200135 (CN)
(74) Representative: Marks & Clerk LLP
(86) International application number: PCT/CN2020/124513
(87) International publication number: WO 2021/098462

(57) **Abstract**

A hemostatic clip and an auxiliary system are disclosed. The hemostatic clip includes a first clip arm (110), a second clip arm (120), a tightening tube (140), a middle clip arm (150), an intermediate assembly (160), a traction assembly (170), a control handle (180) and at least two separators (190). The traction assembly (170) is connected to both the control handle (180) and the intermediate assembly (160), and the intermediate assembly (160) is connected to the tightening tube (140). Both the first clip arm (110) and the second clip arm (120) are proximally connected to the traction assembly (170). The middle clip arm (150) extends through the tightening tube (140) and is fixed proximally to the intermediate assembly (160). The first clip arm (110) and the second clip arm (120) are disposed on opposite sides of the middle clip arm (150). The first clip arm (110) makes up a first clip together with the middle clip arm (150), and the second clip arm (120) makes up a second clip together with the middle clip arm (150). The control handle (180) is configured to open or close the first clip and/or the second clip corresponding to the first clip arm (110) and/or second clip arm (120). The separators (190) are disposed in a lumen of the tightening tube (140) in such a manner that they are individually located between the first clip arm (110) and the middle clip arm (150) and between the second clip arm (120) and the middle clip arm (150), thus reducing the risk of device faults caused by interference of the first clip arm (110) and the second clip arm (120) with the middle clip arm (150).

## Description

### TECHNICAL FIELD

The present invention relates to the field of medical devices and, more particularly, to a hemostatic clip and an auxiliary system.

### BACKGROUND

With the advancement of gastrointestinal endoscopic technology and its increasing application in therapeutic treatment and diagnosis, endoscopic minimally invasive intervention of various benign and malignant gastrointestinal diseases is gaining popularity and finding increasingly extensive use. One of the critical concerns that must be addressed in this field is how to close gastrointestinal tissue defects/perforations during surgery in a safe and effective manner. Currently, endoscopic suturing relies mainly on hemostatic clips and dedicated endoscopic sewing machines, among others.

Commercially available medical devices for closing large or complex tissue defects or perforations include over-the-scope clips (OTSCs) and dedicated endoscopic sewing machines. However, OTSCs are expensive and must be implanted permanently, and the dedicated machines are bulky, associated with complex manipulation and a limited field of view, and also expensive. For these reasons, both these types of products are not popular in China.

Compared to OTSCs and dedicated endoscopic sewing machines, hemostatic clips are being widely used for the closure of large or complex tissue defects or perforations thanks to their advantages including separable clips heads, low cost and ease of manipulation. Such hemostatic clips have three arms: a first clip arm, a second clip arm and a middle clip arm. The first and second clip arms can independently switch between opened and closed positions with respect to the middle clip arm. In a surgical procedure, a tissue defect or perforation can be closed by clamping its one side between the first and middle clip arms and the opposite side between the second and middle clip arms. However, such hemostatic clips are associated with the problem that, following the successive deployment of the first and second clip arms, they tend to interfere with the middle clip arm, which may lead to inseparability of the clip head, device faults, impaired surgical efficiency, or even surgery failures in severe cases.

Therefore, there is an urgent need for improving those conventional hemostatic clips by reducing their risk of device faults caused by interference of the first and second clip arms with the middle clip arm.

### SUMMARY OF THE INVENTION

It is an objective of the present invention to provide a hemostatic clip and an auxiliary system with reduced risk of device faults caused by interference of first and second clip arms with a middle clip arm.

To this end, the hemostatic clip provided in the present invention includes a first clip arm, a second clip arm, a tightening tube, a middle clip arm, an intermediate assembly, a traction assembly, a control handle and at least two separators,
the control handle, the traction assembly, the intermediate assembly and the tightening tube are sequentially connected together along a proximal-to-distal direction, the control handle is connected to a proximal end of the traction assembly, a distal end of the traction assembly is connected to a proximal end of the intermediate assembly, a distal end of the intermediate assembly is connected to the tightening tube,
proximal ends of the first and second clip arms are disposed within the tightening tube and connected to the traction assembly, the middle clip arm is extending through the tightening tube and fixed proximally to the intermediate assembly, first and second clip arms disposed on opposing sides of the middle clip arm, the first clip arm making up a first clip together with the middle clip arm, the second clip arm making up a second clip together with the middle clip arm,
the control handle configured to individually cause the first and second clip arms to move forth and back in the tightening tube by means of the traction assembly, thereby causing distal end(s) of the first clip arm and/or the second clip arm to move toward or away from the middle clip arm and thus opening or closing the first clip and/or the second clip, the separators all disposed within a lumen of the tightening tube in such a manner that at least one of them is located between the first clip arm and the middle clip arm and that at least one of them is located between the second clip arm and the middle clip arm.

Optionally, two separators may be provided in the form of parallel flat sheets.

Optionally, the tightening tube may be provided therein with limiting slots configured to work with the proximal ends of the first and second clip arms to limit proximal movement of the first and second clip arms relative to the tightening tube.

Optionally, limiting protrusions may be provided on sides of the first and second clip arms facing away from a tubular wall of the tightening tube and a stop tab may be provided on a distal end face of the tightening tube. The stop tab may work with the limiting protrusions to limit movement of the first and second clip arms toward a proximal end of the tightening tube.

Optionally, the intermediate assembly may include a sheath-preceding tube, a connecting member and a pull ring attached to the connecting member, the sheath-preceding tube connected proximally to the traction assembly, the sheath-preceding tube detachably connected distally to the tightening tube by the connecting member, the traction assembly configured to cause distal movement of the pull ring, the pull ring configured to separate, as a result of the distal movement thereof, the connecting member from the tightening tube and the sheath-preceding tube, the sheath-preceding tube defining a lumen in which a proximal end of the middle clip arm is accommodated and secured.

Optionally, the connecting member may be a biased member, which is engaged with the tightening tube and the sheath-preceding tube when in a relaxed configuration and is disengaged from the tightening tube and the sheath-preceding tube when in a compressed configuration.

Optionally, the proximal end of the tightening tube may be arranged at the distal end of the sheath-preceding tube. The tightening tube may be provided therein with two insertion holes, and the sheath-preceding tube may be provided therein with limiting holes corresponding to the respective insertion holes. The biased member may be a Ω-shaped spring with opposing end portions, which are inserted through the insertion and limiting holes when the spring is in a relaxed configuration and are removed from the insertion and limiting holes when the spring is in a compressed configuration.

Optionally, the traction assembly may include a sheath, two core wires, two hooks and two coupling pins, a proximal end of the sheath is connected to the control handle, a distal end of the sheath fixedly is connected to the intermediate assembly, the two core wires are disposed within a lumen of the sheath, a proximal end of each core wire is connected to the control handle, a distal end of each core wire is fixedly connected to a respective one of the hooks, the two hooks are connected respectively to the first and second clip arms by the respective coupling pins, the control handle is configured to pull the first and second clip arms forth and back within the tightening tube via the core wires.

Optionally, for each of the hooks, when a force exceeding a predefined value is exerted on its point of engagement with the respective coupling pin, it will be disengaged from the coupling pin.

Optionally, the first clip arm may be provided therein with a first pin hole corresponding to the respective hook, and the second clip arm may be provided therein with a second pin hole corresponding to the respective hook. Prior to use for coupling the hooks to the first and second clip arms, each of the coupling pins may be in the form of a tubular pin with a number of cut slits each extending from one end toward the middle of the pin body. The coupling pins may be used to couple the hooks to the first and second clip arms in such a manner that one of the coupling pins is inserted through both the first pin hole and the respective hook, with its outer diameter being greater at both ends than at the middle.

Optionally, each hook may be flat in shape and define a deformable slot through which one of the coupling pins is inserted.

Optionally, the sheath may include a first proximal sheath branch, a second proximal sheath branch, a distal sheath section and a connecting tube, a proximal end of the connecting tube is fixedly connected to distal ends of the first and second proximal sheath branches, a distal end of the connecting tube is fixedly connected to a proximal end of the distal sheath section, a distal end of the distal sheath section is fixedly connected to the sheath-preceding tube, proximal ends of the first and second proximal sheath branches are fixedly connected to the respective control handles, the two core wires pass sequentially through the distal sheath section, the connecting tube and respectively the first and second proximal sheath branches and connected to the respective control handles.

The auxiliary system provided in the present invention includes an endoscope and the hemostatic clip as defined above. The endoscope is configured for delivery and traction of the hemostatic clip.

The hemostatic clip and the auxiliary system provided in the present invention provide the following benefits:
Disposing the separators in the lumen of the tightening tube so that the separators are individually located between the first and middle clip arms and between the second and middle clip arms separates the first, second and middle clip arms from one another with direct contact therebetween. This avoids interference of the first and second clip arms with the middle clip arm, facilitating advancement and retraction of the first and second clip arms in the tightening tube, and the resulting repeated opening and closing of the first clip made up of the first and middle clip arms and the second clip made up of the second and middle clip arms, under the control of the control handle. Moreover, the middle clip arm can be easily removed from in between the first and second clip arms. In this way, the risk of operational inconvenience caused by interference of the first and second clip arms with the middle clip arm can be reduced.

### BRIEF DESCRIPTION OF THE DRAWINGS

Fig. 1 is a structural schematic showing a hemostatic clip according to a first embodiment of the present invention;
Fig. 2 is a cross-sectional view showing a first clip arm, a second clip arm, a tightening tube, a middle clip arm and an intermediate assembly in the first embodiment of the present invention;
Fig. 3 is a cross-sectional view showing the first clip arm, the second clip arm and the tightening tube in the first embodiment of the present invention;
Fig. 4 is a structural schematic showing the tightening tube in the first embodiment of the present invention;
Fig. 5 is a left view of the tightening tube in the first embodiment of the present invention;
Fig. 6 is a front view of the tightening tube in the first embodiment of the present invention;
Fig. 7 is a structural schematic showing the first and second clip arms being both closed against the middle clip arm in the first embodiment of the present invention;
Fig. 8 is a cross-sectional view showing a clip head and the intermediate assembly in the first embodiment of the present invention;
Fig. 9 a structural schematic showing the clip head and the intermediate assembly in the first embodiment of the present invention;
Fig. 10 is another structural schematic showing the clip head and the intermediate assembly in the first embodiment of the present invention;
Fig. 11 is a structural schematic showing the intermediate assembly in the first embodiment of the present invention;
Fig. 12 is a structural schematic showing a traction assembly in the first embodiment of the present invention;
Fig. 13 is a structural schematic showing a hook in the first embodiment of the present invention;
Fig. 14 is a structural schematic showing the hook and the first clip arm in the first embodiment of the present invention;
Fig. 15 is a structural schematic showing a coupling pin prior to its use for coupling a hook to the first or second clip arm in the first embodiment;
Fig. 16 is a structural schematic showing the coupling pin that has coupled the hook to the first or second clip arm in the first embodiment;
Fig. 17 is a structural schematic showing a control handle in the first embodiment;
Fig. 18 is an exploded view of the control handle in the first embodiment;
Fig. 19 is a structural schematic showing a first clip made up of the first clip arm and the middle clip arm in a closed configuration in the first embodiment of the present invention;
Fig. 20 is a structural schematic showing the first clip made up of the first clip arm and the middle clip arm and a second clip made up of the second clip arm and the middle clip arm both in a closed configuration in the first embodiment of the present invention;
Fig. 21 is a structural schematic showing the hemostatic clip that is undergoing disengagement of hooks from coupling pins and separation of the tightening tube from a sheath-preceding tube in the first embodiment of the present invention;
Fig. 22 is a partial structural schematic showing the hemostatic clip that is undergoing the disengagement of the hooks from the coupling pins and the separation of the tightening tube from the sheath-preceding tube in the first embodiment of the present invention;
Fig. 23 is another partial structural schematic showing the hemostatic clip that is undergoing the disengagement of the hooks from the coupling pins and the separation of the tightening tube from the sheath-preceding tube in the first embodiment of the present invention;
Fig. 24 is a structural schematic showing a hemostatic clip according to a second embodiment;
Fig. 25 is an axonometric view of the hemostatic clip of the second embodiment;
Fig. 26 is a partially enlarged schematic view of the hemostatic clip of the second embodiment;
Fig. 27 is a structural schematic showing the hemostatic clip of the second embodiment;
Fig. 28 is a structural schematic showing a first clip made up of a first clip arm and a middle clip arm in a closed configuration;
Fig. 29 is a structural schematic showing the first clip made up of the first clip arm and the middle clip arm and a second clip made up of a second clip arm and the middle clip arm both in a closed configuration; and
Fig. 30 is a structural schematic showing a clip head that has been separated from an intermediate assembly, a traction assembly and a control handle.

### Reference Numerals in the Drawings

110-First Clip Arm; 120-Second Clip Arm; 130-Limiting Protrusion; 140-Tightening Tube; 141-Limiting Slot; 142-Stop Tab; 143-Insertion Hole; 150-Middle Clip Arm;
160-Intermediate Assembly; 161-Sheath-Preceding Tube; 162-Limiting Hole; 163-Connecting Member; 164-Pull Ring;
170-Traction Assembly; 171-Sheath; 171a-First Proximal Sheath Branch; 171b-Second Proximal Sheath Branch; 171c-Distal Sheath Section; 172-Connecting Tube; 173-Core Wire; 174-Hook; 175-Deformable Slot; 176-Coupling Pin; 177-Cut Slit;
180-Control Handle; 181-Handle Body; 182-Handle Pull Ring; 183-Limiting Nut; 184-Limiting Member; 185-Limiting Pin; 186-Limiting Finger; 187-Limiting Recess; 190-Separator.

### DETAILED DESCRIPTION

The hemostatic clip and auxiliary system proposed in the present invention will be described in greater detail below with reference to the specific embodiments illustrated in the accompanying drawings. Advantages and features of the present invention will become more apparent from the following description and from the appended claims. Note that the drawings are provided in a very simplified form not necessarily drawn to exact scale for the only purpose of facilitating easy and clear description of the disclosed embodiments.

### Embodiment 1

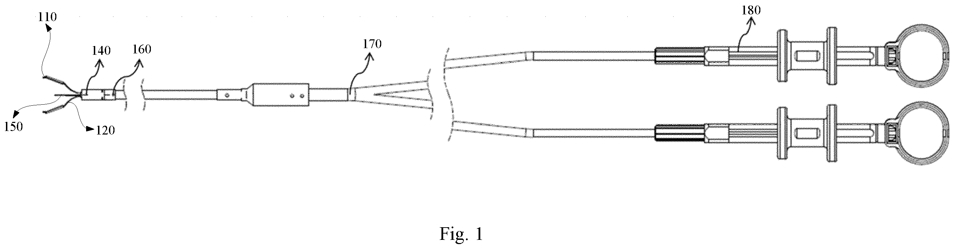

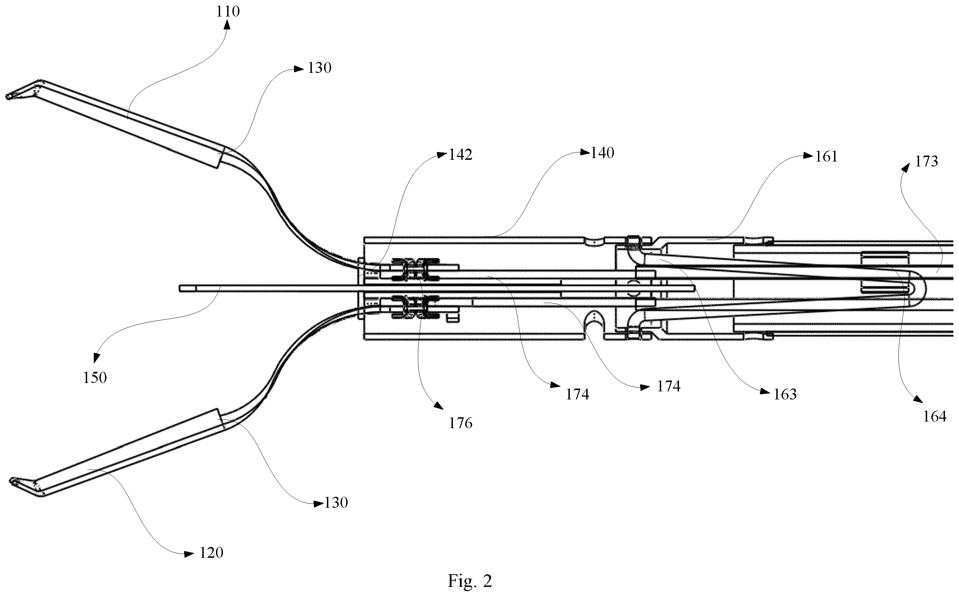

In a first embodiment, there is provided a hemostatic clip. Reference is now made to Figs. 1-2, Fig. 1 is a structural schematic showing the hemostatic clip according to the first embodiment of the present invention, and Fig. 2 is a cross-sectional view illustrating a first clip arm 110, a second clip arm 120, a tightening tube 140, a middle clip arm 150 and an intermediate assembly 160 in the first embodiment of the present invention. The hemostatic clip includes the first clip arm 110, the second clip arm 120, the tightening tube 140, the middle clip arm 150, the intermediate assembly 160, a traction assembly 170 and control handles 180. The control handles 180 are coupled to the traction assembly 170. A distal end of the traction assembly 170 is coupled to a proximal end of the intermediate assembly 160. A distal end of the intermediate assembly 160 is coupled to the tightening tube 140, proximal end portions of the first clip arm 110 and the second clip arm 120 are received in the tightening tube 140. The proximal ends of the first clip arm 110 and the second clip arm 120 are both coupled to the traction assembly 170, and the middle clip arm 150 extends through the tightening tube 140, with its proximal end protruding out of the tightening tube 140 and being fixedly coupled to the intermediate assembly 160. The first clip arm 110 and the second clip arm 120 are arranged on opposing sides of the middle clip arm 150 so that the first clip arm 110 forms a first clip with the middle clip arm 150 and the second clip arm 120 forms a second clip with the middle clip arm 150. The control handles 180 are configured to individually control the first clip arm 110 and the second clip arm 120 by means of the traction assembly 170 to move forth or back within the tightening tube 140 and thereby individually open or close the first and second clips.

Specifically, the movement of the first clip arm 110 and the second clip arm 120 forth or back within the tightening tube 140 means that the first clip arm 110 and the second clip arm 120 move from the proximal end to the distal end, or move from the distal end to the proximal end, within the tightening tube 140.

As a result of the first clip arm 110 moving in the tightening tube 140 toward the distal end thereof, a distal end of the first clip arm 110 moves away from the middle clip arm 150, thus opening the first clip made up of the first clip arm 110 and the middle clip arm 150. As a result of the first clip arm 110 moving in the tightening tube 140 toward the proximal end thereof, the distal end of the first clip arm 110 approaches the middle clip arm 150, closing the first clip made up of the first clip arm 110 and the middle clip arm 150. Similarly, as a result of the second clip arm 120 moving in the tightening tube 140 toward the distal end thereof, a distal end of the second clip arm 120 moves away from the middle clip arm 150, opening the second clip made up of the second clip arm 120 and the middle clip arm 150. As a result of the second clip arm 120 moving in the tightening tube 140 toward the proximal end thereof, the distal end of the second clip arm 120 approaches the middle clip arm 150, closing the second clip made up of the second clip arm 120 and the middle clip arm 150. That is to say, in this embodiment, distal movement of any of the first clip arm 110 and the second clip arm 120 within the tightening tube 140 will open a corresponding one of the first and second clips that correspond to the first clip arm 110 and the second clip arm 120, respectively, and proximal movement of any of the first clip arm 110 and the second clip arm 120 within the tightening tube 140 will close a respective one of the first and second clips that are formed by the first clip arm 110 and the second clip arm 120 with the middle clip arm 150, respectively.

The middle clip arm 150 serves mainly for anchoring and supporting.

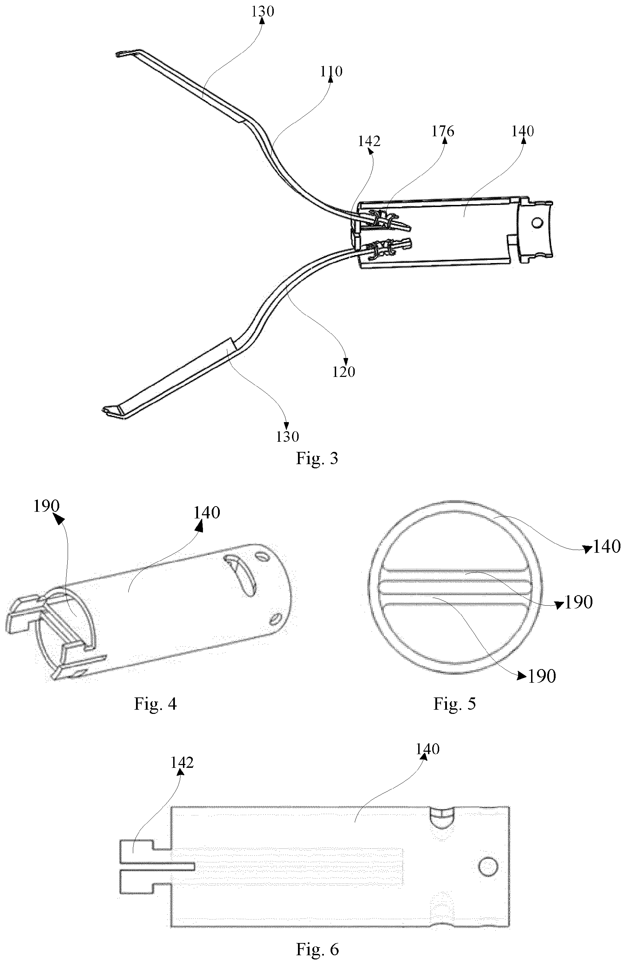

Reference is now made to Figs. 3, 4, 5 and 6, Fig. 3 is a cross-sectional view of the first clip arm 110, the second clip arm 120 and the tightening tube 140 in the first embodiment of the present invention, Fig. 4 is a structural schematic showing the tightening tube 140 in the first embodiment of the present invention, Fig. 5 is a left view of the tightening tube 140 in the first embodiment of the present invention, and Fig. 6 is a front view of the tightening tube 140 in the first embodiment of the present invention. The hemostatic clip further includes at least two separators 190. The separators 190 are disposed in a lumen of the tightening tube 140 so as to be positioned respectively between the first clip arm 110 and the middle clip arm 150 and between the second clip arm 120 and the middle clip arm 150.

Disposing the separators 190 in the lumen of the tightening tube 140 and positioning the separators 190 respectively between the first clip arm 110 and the middle clip arm 150 and between the second clip arm 120 and the middle clip arm 150 spaces the first clip arm 110, the second clip arm 120 and the middle clip arm 150 apart without direct contact, addressing the problem of unsuccessful withdrawal of the middle clip arm due to direct contact between and mutual interference of the first and second clip arms, as seen in the prior art. As a result, the first clip arm 110 and the second clip arm 120 can be more easily moved forth and back within the tightening tube 140 under the control of the control handles 180, causing the first clip made up of the first clip arm 110 and the middle clip arm 150 and the second clip made up of the second clip arm 120 and the middle clip arm 150 to be repeatedly opened and closed, and the middle clip arm 150 can be more easily withdrawn from in between the first clip arm 110 and the second clip arm 120 with less interference of the first 110 or second 120 arm with the middle clip arm 150.

Specifically, in the examples shown in Figs. 3, 4, 5 and 6, two separators 190 are provided in the form of two parallel plates each fixedly attached at opposing sides to a tubular wall of the tightening tube 140. In other examples, each separator 190 may be fixedly attached at one side to the tubular wall of the tightening tube 140, with the other side of the separator 190 being free. That is to say, as long as the first clip arm 110, the second clip arm 120 and the middle clip arm 150 are provided with the separators 190 which may completely separate the first clip arm 110 from the middle clip arm 150 and the second clip arm 120 and the middle clip arm 150, or partially separate them.

In other embodiments, even more separators 190 may be provided. For example, four separators 190 may be provided, each fixedly attached at one side to the tubular wall of the tightening tube 140, with the other side of the separator 190 being a free side not attached to the tubular wall of the tightening tube 140. Moreover, the separators 190 may be grouped into two pairs, one of which spaces the first clip arm 110 apart from the middle clip arm 150, and the other of the separator 190 spaces the second clip arm 120 apart from the middle clip arm 150. In such embodiments, the separators 190 may be ribs or ridges in the form of plates extending both along an axis of the tightening tube 140 and inwardly from the tubular wall of the tightening tube 140. As another example, three separators 190 may be provided, each fixedly attached to the tubular wall of the tightening tube 140 at one side and being free and not attached to the tubular wall of the tightening tube 140 at the other side. Moreover, two of the separators 190 may form a pair that spaces the first clip arm 110 apart from the middle clip arm 150, with the remaining separator 190 spacing the second clip arm 120 apart from the middle clip arm 150. Each of the three separators 190 may be a rib or ridge in the form of a plate extending both inwardly from the tubular wall of the tightening tube 140 and along an axis of the tightening tube 140. Alternatively, the one out of the three separators 190 that spaces the second clip arm 120 apart from the middle clip arm 150 may be attached at both sides to the tubular wall of the tightening tube 140.

In this embodiment, each separator 190 is a plate, both sides of each separator 190 are fixedly attached to the tubular wall of the tightening tube 140, and the first clip arm 110 is entirely separated from the middle clip arm 150 and the second clip arm 120 is entirely separated from the middle clip arm 150. In the implementations where each separator 190 is fixedly attached to the tubular wall of the tightening tube 140 and free at the other side, the first clip arm 110 can be partially separated from the middle clip arm 150 and the second clip arm 120 can be partially separated from the middle clip arm 150. Although entire separation of the first clip arm 110 and the second clip arm 120 from the middle clip arm 150 is not attained by the separators 190, gaps between the first clip arm 110 and the second clip arm 120 and the middle clip arm 150 resulting from thicknesses of the separators 190 can still avoid interference of the first clip arm 110 and the second clip arm 120 with the middle clip arm 150.

The separators 190 are arranged along the axis of the tightening tube 140. It is possible either that the separators 190 are arranged so as to be spaced apart along the axis of the tightening tube 140, or that the separators 190 each extend throughout the tightening tube 140 along the axis thereof, as long as the separators 190 can separate the first clip arm 110 from the middle clip arm 150 and the second clip arm 120 from the middle clip arm 150. In other words, the separators 190 may be either continuous or non-continuous along the axis of the tightening tube 140. The separators 190 may have different lengths along the axis of the tightening tube 140.

Fig. 6 shows the separators 190 being axially shorter than the tightening tube 140 in order to facilitate the making of the tightening tube 140 as well as its attachment and coupling to the separators 190 and the intermediate assembly 160.

In this embodiment, the tightening tube 140 and the separators 190 may be either integrally formed, or welded together. The tightening tube 140 and the separators 190 may also be made from at least two metal sheets by folding and welding.

In this embodiment, the separators 190 are preferably slidably attached to the first clip arm 110, the second clip arm 120 and the middle clip arm 150. This can avoid interference of the first clip arm 110 and the second clip arm 120 with the middle clip arm 150, so that the repeated movement of the first clip arm 110, the middle clip arm 150 and the second clip arm 120 can be smoother, resulting in improved user experience and increased surgical efficiency.

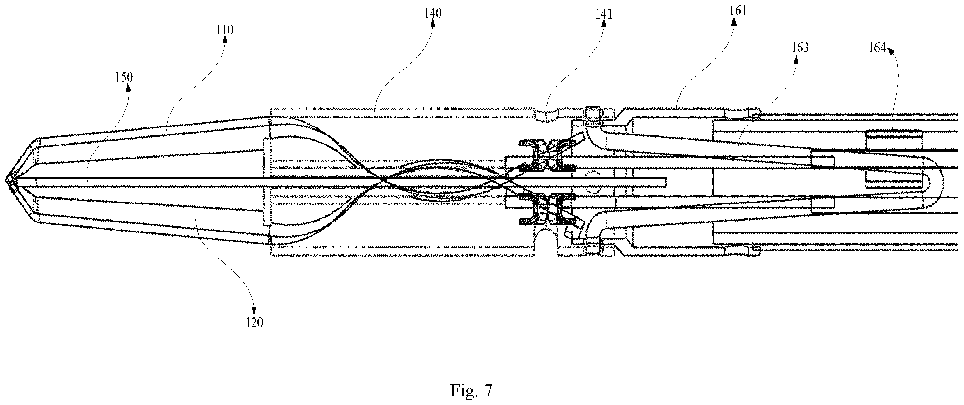

Reference is made back to Figs. 4 and 7, Fig. 7 is a structural schematic showing the first clip arm 110 and the second clip arm 120 being both closed against the middle clip arm 150 in the first embodiment of the present invention. The tightening tube 140 defines limiting slots 141. Specifically, when the proximal end of any of the first clip arm 110 and the second clip arm 120 moves over a respective one of the limiting slots 141, under the compression of the tightening tube 140, the proximal end of the first clip arm 110 or the second clip arm 120 will extend into the limiting slot 141. As a result, further proximal movement of the first clip arm 110 or the second clip arm 120 is limited due to interaction of its proximal end with the limiting slot 141, avoiding the first clip arm 110 or the second clip arm 120 from protruding out of a proximal end of the tightening tube 140. This can improve reliability of the hemostatic clip by reducing the risk of the first clip arm 110 or the second clip arm 120 protruding beyond the proximal end of the tightening tube 140. The interaction of the proximal end of the first clip arm 110 or the second clip arm 120 with the limiting slot 141 may be entrapping of the proximal end in the limiting slot 141.

In order to more reliably avoid the first or second clip arm from protruding beyond the proximal end of the tightening tube, on the sides of the first clip arm 110 and the second clip arm 120 facing away from the tubular wall of the tightening tube 140, respective limiting protrusions 130 are provided, which provide an additional limiting effect. Specifically, referring to Figs. 2, 3, 4 and 7, a stop tab 142, against which the limiting protrusions 130 can abut, is provided on a distal end face of the tightening tube 140. The abutment of any of the limiting protrusion 130 against the stop tab 142 can limit further movement of the corresponding first clip arm 110 or the second clip arm 120 toward the proximal end of the tightening tube 140. As a result, the first clip arm 110 or the second clip arm 120 can be more reliably avoided from protruding beyond the proximal end of the tightening tube 140, resulting in an increase in the reliability of the hemostatic clip. Specifically, in addition to the interaction of the proximal end of any of the first clip arm 110 and the second clip arm 120 with a respective one of the limiting slots 141 occurring as a result of movement of the proximal end over the limiting slot 141, the limiting protrusions 130 can additionally limit proximal movement of the first clip arm 110 and the second clip arm 120 and thus avoid the first clip arm 110 and the second clip arm 120 from protruding out of the proximal end of the tightening tube 140. This double limiting design can reduce the risk of the first clip arm 110 or the second clip arm 120 protruding out of the proximal end of the tightening tube 140.

In other embodiments, the limiting protrusions 130 may be alternatively provided on the respective sides of the first clip arm 110 and the second clip arm 120 facing toward the tubular wall of the tightening tube 140, as long as when any of the first clip arm 110 and the second clip arm 120 is moving toward the proximal end of the tightening tube 140, its further proximal movement is limited when a respective one of the limiting protrusions 130 comes into abutment against the distal end of the tightening tube 140 and/or the stop tab 142 disposed at the distal end of the tightening tube 140.

The tightening tube 140 has an outer diameter of 2.2-2.6 mm and a length of 5-8 mm. Each of the first clip arm 110, the second clip arm 120 and the tightening tube 140 may be made of a titanium alloy or stainless steel. Each of the first clip arm 110 and the second clip arm 120 is flared away from the middle clip arm 150 at an angle of 15-75 °.

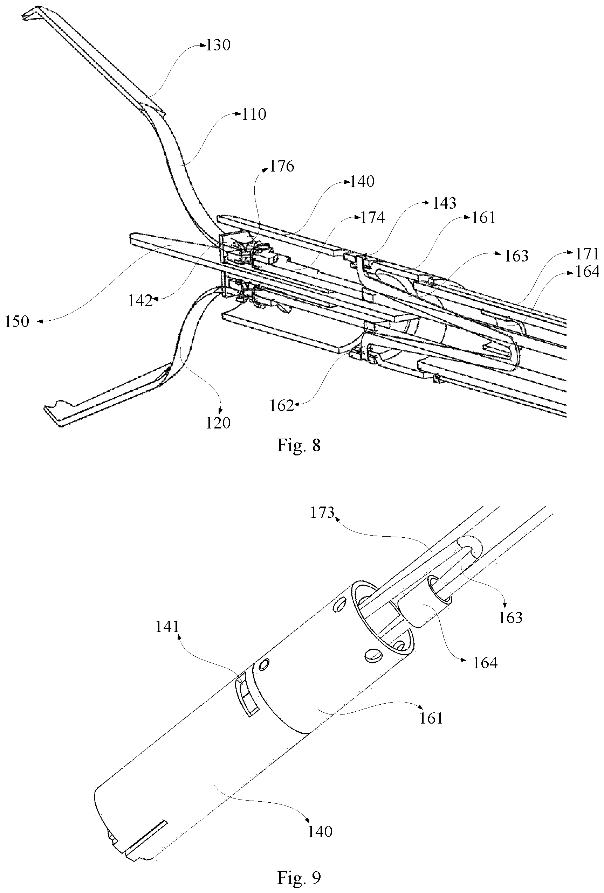

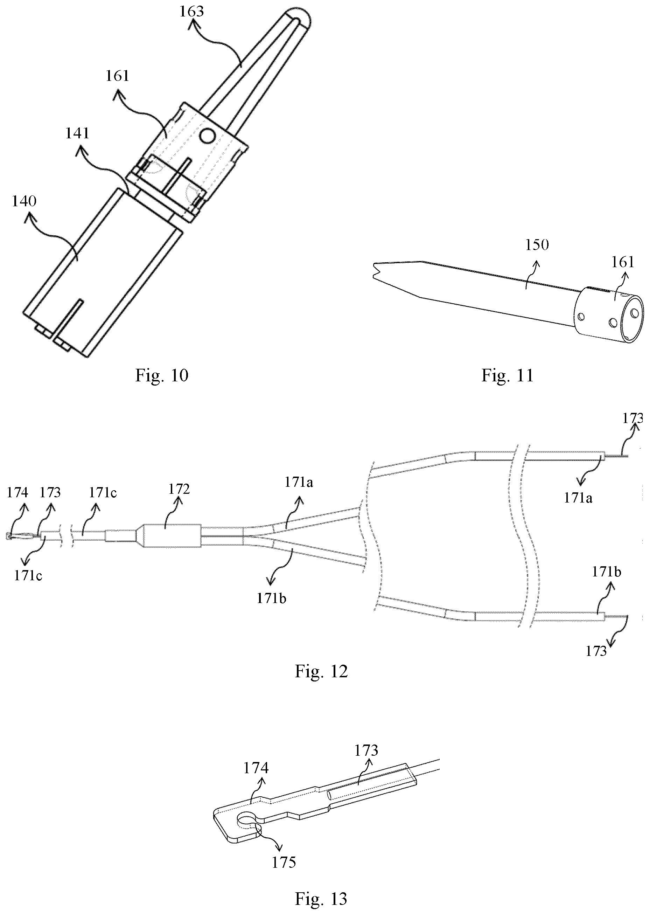

Reference is now made to Figs. 8, 9, 10 and 11. Fig. 8 is a cross-sectional view showing a clip head and the intermediate assembly 160 in the hemostatic clip of the first embodiment of the present invention. The clip head in the hemostatic clip is made up of the tightening tube 140, the first clip arm 110 and the second clip arm 120. Fig. 9 is a structural schematic showing the clip head and the intermediate assembly 160 in the first embodiment of the present invention. Fig. 10 is another structural schematic showing the clip head and the intermediate assembly 160 in the first embodiment of the present invention. Fig. 11 is a structural schematic showing the intermediate assembly 160 in the first embodiment of the present invention. The intermediate assembly 160 includes a sheath-preceding tube 161, a connecting member 163 and a pull ring 164 attached to the connecting member 163.

A proximal end of the sheath-preceding tube 161 is coupled to the traction assembly 170 (see Fig. 1), and a distal end of the sheath-preceding tube 161 is detachably coupled to the tightening tube 140 via the connecting member 163. The traction assembly 170 is configured to drive distal movement of the pull ring 164, the distal movement of the pull ring 164 causes separation of the connecting member 163 from the tightening tube 140 and the sheath-preceding tube 161. A proximal end of the middle clip arm 150 is disposed and fixed in a lumen of the sheath-preceding tube 161. This allows the traction assembly 170 to drive the pull ring 164 to move distally, and the distal movement in turn causes separation of the connecting member 163 from the sheath-preceding tube 161 and the tightening tube 140 and subsequent separation of the sheath-preceding tube 161 from the tightening tube 140. The separation of the sheath-preceding tube 161 from the tightening tube 140 follows successful clamping of target tissue by the first clip arm 110 and the second clip arm 120. At this point, when the traction assembly 170 is manipulated to cause the intermediate assembly 160 to move proximally, the intermediate assembly 160 will be separated from the clip head, followed by the separation of the middle clip arm 150 from the first clip arm 110 and the second clip arm 120.

The connecting member 163 is a biased member, when in a relaxed configuration, the connecting member 163 is attached to both the tightening tube 140 and the sheath-preceding tube 161, thereby coupling the tightening tube 140 to the sheath-preceding tube 161. The biased member may be compressed and the connecting member 163 is detached from the tightening tube 140 and the sheath-preceding tube 161, thus allowing the tightening tube 140 to be separated from the sheath-preceding tube 161.

As shown in Fig. 8, a proximal end portion of the tightening tube 140 is sleeved on a distal end portion of the sheath-preceding tube 161. The tightening tube 140 is provided with two insertion holes 143 and the sheath-preceding tube 161 with limiting holes 162 corresponding to the respective insertion holes 143. The biased member is a Ω-shaped spring, whose opposing ends are inserted in the insertion holes 143 and the limiting holes 162 in the relaxed configuration of the spring, limiting axial movement of the tightening tube 140 relative to the sheath-preceding tube 161, in particular, limiting the tightening tube 140 and the sheath-preceding tube 161 from being separated from each other in the axial direction. In the compressed configuration of the spring, its opposing ends are withdrawn from the insertion holes 143 and the limiting holes 162.

The Ω-shaped spring may be formed by bending a piece of metal such as stainless steel. The pull ring 164 may be either a metal ring or a plastic ring.

The sheath-preceding tube 161 may be fixed to the middle clip arm 150 by welding. It may be also fixed to the traction assembly 170 by welding. The sheath-preceding tube 161 may be a metal tube, such as a stainless steel tube.

The sheath-preceding tube 161 has a length of 3-8 mm and may be made of stainless steel or a titanium alloy.

Referring to Fig. 11, the middle clip arm 150 is flat in shape and may be fabricated by cutting a metal sheet.

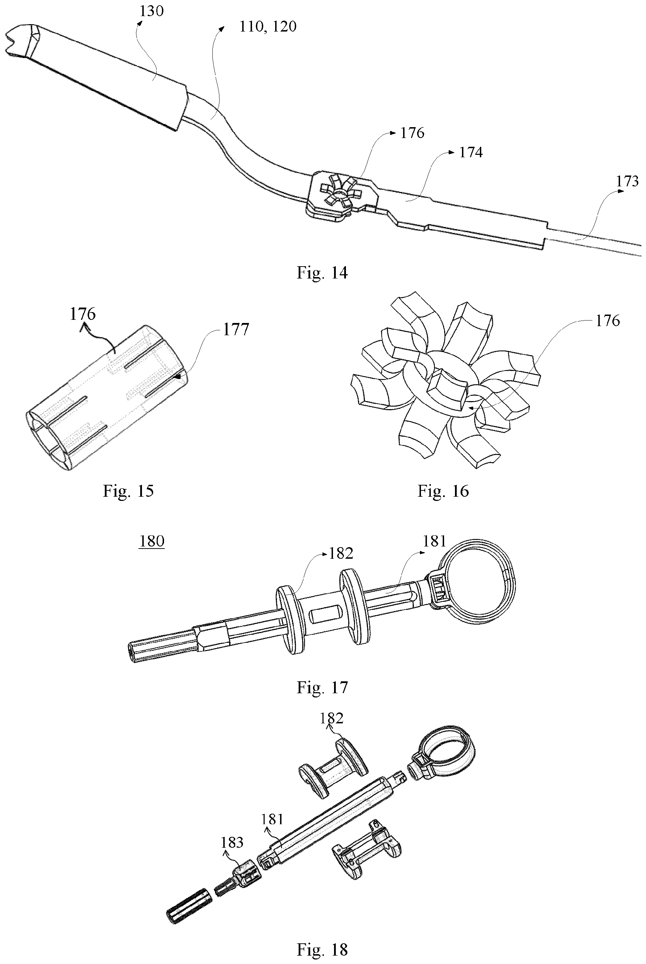

Reference is now made to Figs. 12, 13 and 14, Fig. 12 is a structural schematic showing the traction assembly 170 in the first embodiment of the present invention, Fig. 13 is a structural schematic showing a hook 174 in the first embodiment of the present invention, and Fig. 14 is a structural schematic showing the hook 174 and the first clip arm 110 in the first embodiment of the present invention. The traction assembly 170 includes a sheath 171, two core wires 173, two hooks 174 and two coupling pins 176. A proximal end of the sheath 171 is coupled to the control handles 180, and a distal end of the sheath 171 is coupled to the intermediate assembly 160 (more precisely, to the distal end of the sheath-preceding tube 161). The two core wires 173 are both disposed within a lumen of the sheath 171 so as to be coupled proximally to the control handles 180 and fixed distally to the respective hooks 174. The two hooks 174 are respectively coupled to the first clip arm 110 and the second clip arm 120 by the respective coupling pins 176. The control handles 180 are provided to pull the first clip arm 110 and the second clip arm 120 individually via the two core wires 173 to cause them move forth or back in the tightening tube 140, thereby opening or closing the first clip made up of the first clip arm 110 and the middle clip arm 150 and the second clip made up of the second clip arm 120 and the middle clip arm 150. For each of the hooks 174, if a force exceeding a predefined value is exerted on a point of engagement of the hook 174 with the respective coupling pin 176, it will be disengaged from the coupling pin 176.

When the engagement between any hook 174 and the respective coupling pin 176 is eliminated by a force exceeding the predefined value exerted on a point of engagement between the hook 174 and the respective coupling pin 176, the indirect coupling between the hook 174 and the first 110 or second 120 arm established by the coupling pin 176 will fail, allowing separation of the traction assembly 170 from the first clip arm 110 or the second clip arm 120. When the traction assembly 170 is separable from both the first clip arm 110 and the second clip arm 120, the intermediate assembly 160 is separable from the tightening tube, the first clip arm 110, the second clip arm 120 and the tightening tube 140 become separable from the middle clip arm 150, the intermediate assembly 160, the traction assembly 170 and the control handles 180.

Specifically, if any hook 174 is disengaged from the respective coupling pin 176 as a result of a force exceeding the predefined value exerted on a point of engagement therebetween, the coupling between the hook 174 and the first clip arm 110 or second clip arm 120 will be disconnected.

Reference is now made to Figs. 15 and 16, Fig. 15 is a structural schematic showing one of the coupling pins 176 prior to its use for coupling one of the hooks 174 to the first clip arm 110 or the second clip arm 120 in the first embodiment, and Fig. 16 is a structural schematic showing the coupling pin 176 that has coupled the hook 174 to the first clip arm 110 or the second clip arm 120 in the first embodiment. The first clip arm 110 is provided with a first pin hole for receiving one of the coupling pins 176, and the second clip arm 120 is provided with a second pin hole for receiving the other of the coupling pins 176. Before being used to couple the hooks 174 to the first clip arm 110 and the second clip arm 120, each of the coupling pins 176 is provided as a tubular pin with a number of cut slits 177 therein each extending from one end toward the middle of the pin body. The coupling pins 176 are used to couple the hooks 174 to the first clip arm 110 and the second clip arm 120 in such a manner that one of the coupling pins 176 is inserted through both the first pin hole and the respective hook 174, the outer diameter of both ends of the coupling pin 176 is greater than the outer diameter of the middle portion of the coupling pin 176.

In order to couple one of the hooks 174 to the first clip arm 110 by one of the coupling pins 176, the coupling pin 176 is inserted through both the first pin hole and the hook 174, and the other coupling pin 176 is inserted through both the second pin hole and the other hook 174. The two coupling pins 176 are then pressed at both ends so that end portions of each coupling pin 176 are folded away from a central line of the coupling pin 176, so that the outer diameter of both ends of the coupling pin 176 is greater than the outer diameter of the middle portion of the coupling pin 176. As a result, the first clip arm 110 and one hook 174 are confined between the folded end portions of one coupling pin 176 and cannot move away from each other, and the second clip arm 120 and the other hook 174 are confined between the folded end portions of the other coupling pin 176 and cannot move away from each other. In this way, the first clip arm 110 is coupled to one hook 174 by one coupling pin 176, and the second clip arm 120 is coupled to the other hook 174 by the other coupling pin 176.

Specifically, before being used to couple the hooks 174 to the first clip arm 110 and the second clip arm 120, each of the coupling pins 176 has an axial length in the range of 2-3 mm. The coupling pins 176 are stainless steel tubes which may be formed by laser cutting. The slits 177 in the stainless steel tubes may also be formed by laser cutting. The coupling pins 176 are easy to fabricate, convenient to manipulate, and highly reliable.

Referring to Figs. 13 and 14, each hook 174 is flat and defines a deformable slot 175, through which one of the coupling pins 176 is inserted. The deformable slot 175 may deform under the action of a force exceeding the predefined value exerted on a point of engagement between the hook 174 and the coupling pin 176, thus allowing disengagement of the hook 174 from the coupling pin 176.

The hooks 174 are fixed to the core wires 173 by laser welding.

Referring to Figs. 9 and 10, the pull ring 164 is received in the metal tube preceding the sheath 171, and both one of the core wires 173 and one leg of the biased member are inserted through the pull ring 164. The core wires 173 are made of stainless steel or a titanium alloy.

Referring to Fig. 12, the sheath 171 includes a first proximal sheath branch 171a, a second proximal sheath branch 171b, a distal sheath section 171c and a connecting tube 172. A proximal end of the connecting tube 172 is fixed to distal ends of both the first and second proximal sheath branches 171a, 171b, and a distal end of the connecting tube 172 is fixed to a proximal end of the distal sheath section 171c. A distal end of the distal sheath section 171c is fixed to the sheath-preceding tube 161, and proximal ends of the first and second proximal sheath branches 171a, 171b are fixed to the respective control handles 180. The two core wires 173 are passed sequentially through the distal sheath section 171c, the connecting tube 172 and respectively the first and second proximal sheath branches 171a, 171b and coupled to the respective control handles 180. In this way, the two control handles 180 can be manipulated to individually control the first clip arm 110 and the second clip arm 120, resulting in more flexibility in use of the device. Further, the two control handles 180 can be operated by different persons. This lowers the requirements on the dexterity of physicians involved in endoscopic surgery, reduced the difficulties associated with such surgical procedures and facilitates wider application of cutting-edge endoscopic surgical techniques.

The fixation of the sheath-preceding tube 161 to the distal end of the distal sheath section 171c may be accomplished by laser welding. The fixation of the distal end of the connecting tube 172 to the proximal end of the distal sheath section 171c may be accomplished by welding. The fixation of the proximal end of the connecting tube 172 to the first and second proximal sheath branches 171a, 171b may be accomplished also by welding.

Outer diameters of the first proximal sheath branch 171a, the second proximal sheath branch 171b, the distal sheath section 171c and the connecting tube 172 range from 2 mm to 2.4 mm. The first and second proximal sheath branches 171a, 171b both have a length in the range of 50-150 mm. A length of the distal sheath section 171c is 120-250 mm. A length of the connecting tube 172 is 10-30 mm.

Reference is now made to Figs. 17 and 18, Fig. 17 is a structural schematic showing one of the control handles 180 in the first embodiment, and Fig. 18 is an exploded view of the control handle 180 in the first embodiment. The first and second proximal sheath branches 171a, 171b are fixed to the respective control handles. Each control handle 180 includes a handle body 181, a handle pull rings 182 and a limiting nut 183. The handle pull rings 182 is sleeved on the handle body 181 so as to be movable forth and back relative to the handle body 181. The limiting nut 183 is mated with a distal end of the handle body 181. The first and second proximal sheath branches 171a, 171b are fixed to the respective limiting nuts 183 and the respective handle bodies 181. Each handle body 181 defines a control socket that is open distally, and each limiting nut 183 defines a limiting bore that is open both proximally and distally. The limiting bore is brought at the proximal end thereof into communication with the control socket. One of the core wires 173 is passed sequentially through the limiting bore and the control socket and fixed to the handle pull rings 182. The handle pull rings 182 may be manipulated to move forth and back relative to the handle body 181 together with the core wire 173, thus driving the first clip arm 110 or the second clip arm 120 to also move forth and back.

Specifically, the traction assembly further includes limiting rings, to which the respective proximal ends of the first and second proximal sheath branches 171a, 171b are fixed. Each limiting nut 183 defines a ring-shaped limiting member in the limiting bore. The two limiting rings to which the first and second proximal sheath branches 171a, 171b are fixed are disposed within the respective limiting bores so that the respective limiting members therein can limit distal movement of the limiting rings relative to the limiting nuts 183. In addition, limiting pins extend from the distal ends of the handle bodes into the respective limiting bores in order to limit proximal movement of the respective limiting rings relative to the limiting nuts 183. Limited by both the limiting members and the limiting pins, the limiting rings can move only within the limiting bores of the respective limiting nuts 183. As a result, movement of the first and second proximal sheath branches 171a, 171b that are fixed to the limiting rings are also limited. The limiting rings may be each made of a metal.

The proximal ends of the limiting nuts 183 are extended with limiting fingers. The handle bodies 181 are provided with limiting recesses which are mated with the limiting fingers. The limiting nuts 183 are fixedly connected to the handle bodies 181 by snapped engagement between the limiting fingers and the limiting recesses.

The control handles 180 are formed of ABS plastic.

In this embodiment, the first clip arm 110 and the second clip arm 120 can be independently opened and closed repeatedly.

Specifically, since the two control handles 180 are independent of each other and operated in a similar manner to respectively cause the first clip arm 110 and the second clip arm 120 to be opened and closed, only the repeated opening and closing of the first clip arm 110 achieved by a respective one of the control handles 180 is explained below as an example.

When the handle pull rings 182 of the control handle 180 is urged to move proximally relative to the handle body 181, the core wire 173 will be retracted via the handle pull rings 182 to also move proximally relative to the handle body 181. As a result, the first clip arm 110 is retracted by the core wire 173 to move proximally, concurrently with its distal approaching the middle clip arm 150. Finally, the first clip made up of the first clip arm 110 and the middle clip arm 150 is closed. Specifically, proximally driving the handle pull rings 182 may cause proximal movement of the core wire 173, the hook 174 and the coupling pin 176, and the proximal movement of the coupling pin 176 may in turn cause proximal movement of the first clip arm 110. Before the proximal end of the first clip arm 110 comes into engagement with the limiting slot 141 in the tightening tube 140 as a result of the proximal movement of the handle pull rings 182 relative to the handle body 181, the handle pull rings 182 may be always caused to move distally relative to the handle body 181 to cause the distal end of the first clip arm 110 to move away from the middle clip arm 150, thus opening the first clip made up of the first clip arm 110 and the middle clip arm 150. In this way, during use of the hemostatic clip in a surgical procedure, the handle pull rings 182 may be manipulated as required to move proximally or distally relative to the handle body 181 to close or open the first clip made up of the first clip arm 110 and the middle clip arm 150, with improved operability of the hemostatic clip, enhanced convenience for the operating physician and increased surgical efficiency.

In this embodiment, the first clip arm 110, the second clip arm 120 and the tightening tube 140 can be separated from the intermediate assembly 160 and the traction assembly 170. The first clip arm 110, the second clip arm 120 and the tightening tube 140 make up the clip head of the hemostatic clip.

At first, both the first clip arm 110 and the second clip arm 120 may be closed by manipulating the two control handles 180. Specifically, since the two control handles 180 are independent of each other and operated in a similar manner to respectively close the first clip arm 110 and the second clip arm 120 only the closure of the first clip arm 110 achieved by a respective one of the control handles 180 is explained below as an example.

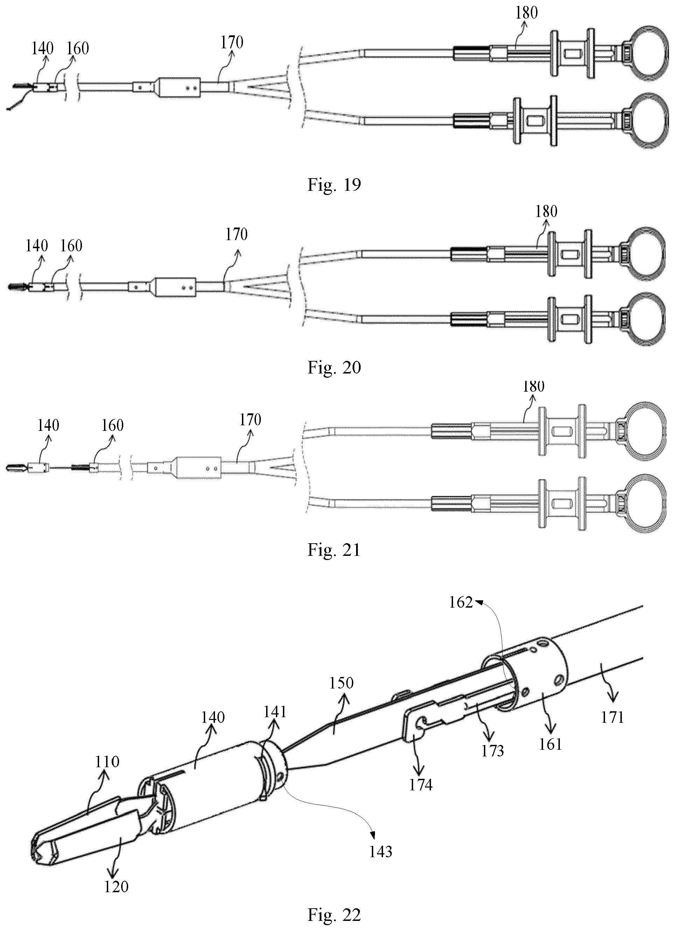

When the handle pull rings 182 is urged to move proximally relative to the handle body 181, the core wire 173 will be driven by the handle pull rings 182 to move proximally relative to the handle body 181, causing the first clip arm 110 to move proximally relative to the handle body 181 concurrently with its distal end approaching the middle clip arm 150. As a result, the first clip made up of the first clip arm 110 and the middle clip arm 150 is closed. When the first clip arm 110 moves into engagement at the proximal end with the limiting slot 141, the limiting slot 141 limits the further proximal movement of the first clip arm 110. At this point, the first clip made up of the first clip arm 110 and the middle clip arm 150 is in the closed configuration, as shown in Fig. 19. Fig. 19 is a structural schematic showing the first clip made up of the first clip arm 110 and the middle clip arm 150 in the closed configuration in the first embodiment of the present invention. The first clip made up of the first clip arm 110 and the middle clip arm 150 and the second clip made up of the second clip arm 120 and the middle clip arm 150, both of which are in a closed configuration are shown in Fig. 20. Fig. 20 is a structural schematic showing the first clip made up of the first clip arm 110 and the middle clip arm 150 and the second clip made up of the second clip arm 120 and the middle clip arm 150, both of which are in a closed configuration in the first embodiment of the present invention.

Subsequently, when the handle pull rings 182 are further manipulated to apply pulling forces exceeding the aforementioned predefined value respectively to the junction of the first clip arm 110 and the respective core wire 173 and the junction of the second clip arm 120 and the respective core wire 173, via the respective core wires 173, the coupling of the first clip arm 110 and the respective core wire 173 and the coupling of the second clip arm 120 and the respective core wire 173 will be eliminated. In this process, the pulling forces exceeding the predefined value exerted on the junctions of the first clip arm 110 and the second clip arm 120 deform the deformable slots 175 of the hooks 174, thereby disengaging the hooks 174 from the coupling pins 176. As a result, the first clip arm 110 and the second clip arm 120 are disconnected from the traction assembly 170.

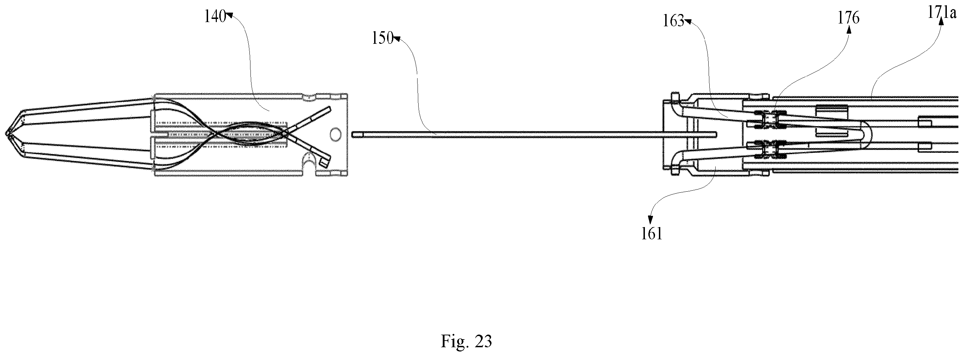

After that, when the handle pull rings 182 are further manipulated to cause further proximal movement of the core wires 173 and the hooks 174, the core wires 173 will cause proximal movement of the pull ring 164, which will lead to separation of the connecting member 163 from the tightening tube 140 and the sheath-preceding tube 161. Specifically, since both one of the core wires 173 and one leg of the biased member are inserted through the pull ring 164, after the hooks 174 have been disengaged from the first clip arm 110 and the second clip arm 120, further pulling the core wires 173 will cause proximal movement of the pull ring 164 together with the core wires 173 and hooks 174. As a result of the proximal movement of the pull ring 164, the opposing ends of the connecting member 163 will move toward each other and dislodge from the tightening tube 140 and the sheath-preceding tube 161, thus unlocking the connecting member 163 from the tightening tube 140 and sheath-preceding tube 161. Reference can be made to Figs. 21, 22 and 23 for the disengagement of the hooks 174 from the coupling pins 176 as well as for the separation of the tightening tube 140 from the sheath-preceding tube 161. Fig. 21 is a structural schematic showing the hemostatic clip that is undergoing the disengagement of the hooks 174 from the coupling pins 176 and the separation of the tightening tube 140 from the sheath-preceding tube 161 in the first embodiment of the present invention. Fig. 22 a partial structural schematic showing the hemostatic clip that is undergoing the disengagement of the hooks 174 from the coupling pins 176 and the separation of the tightening tube 140 from the sheath-preceding tube 161 in the first embodiment of the present invention. Fig. 23 is another partial structural schematic showing the hemostatic clip that is undergoing the disengagement of the hooks 174 from the coupling pins 176 and the separation of the tightening tube 140 from the sheath-preceding tube 161 in the first embodiment of the present invention.

Since disconnection of the core wires 173 from the first clip arm 110 and the second clip arm 120 and separation of the tightening tube 140 from the connecting member 163 and the sheath-preceding tube 161 successively occurs as a result of pulling the handle pull rings 182 proximally relative to the relative to handle body 181, the first clip arm 110, the second clip arm 120 and the tightening tube 140 can be separated from the traction assembly 170 and the intermediate assembly 160. The separation of the first clip arm 110, the second clip arm 120 and the tightening tube 140 from the traction assembly 170 and the core wires 173 enables another device to adjust the position of the separated clip head during surgery. Since the coupling pins 176 that have been disengaged from the hooks 174 still remain attached respectively to the first clip arm 110 or the second clip arm 120, they are separated from the traction assembly 170 and the intermediate assembly 160 together with the first clip arm 110, the second clip arm 120 and the tightening tube 140.

In this embodiment, there is also provided an auxiliary system incorporating the above hemostatic clip and an endoscope configured for delivery and traction of the hemostatic clip.

In this embodiment, a process for closing a tissue defect using the auxiliary system essentially includes the steps detailed below.

At first, the tissue defect is identified by using the endoscope.

Next, the hemostatic clip is delivered to the tissue defect, and the first clip arm 110 and the second clip arm 120 of the hemostatic clip are released by operating a clamp channel of the endoscope. The released first and second clip arms 110, 120 of the hemostatic clip are in the open configurations.

Subsequently, the lesion is clamped by the first clip arm 110, the second clip arm 120 and the middle clip arm 150 by repeatedly opening and closing the first clip arm 110 and the second clip arm 120 of the hemostatic clip. In order to clamp the lesion by the clip head, one of the control handles 180 may be manipulated to position one side of the tissue defect between a respective one of the first clip arm 110 and the second clip arm 120 (e.g., the first clip arm 110) and the middle clip arm 150, close the first clip arm 110 against the middle clip arm 150, and separate both the first clip arm 110 and the respective coupling pin 176 from the respective hook 174. After that, the opposite side of the tissue defect is positioned between the second clip arm 120 and the middle clip arm 150 as a result of pulling actions of the endoscope, and the other control handle 180 is manipulated in a similar way to close the second clip arm 120 against the middle clip arm 150 and separate both the second clip arm 120 and the respective coupling pin 176 from the respective hook 174.

Afterwards, the two control handles 180 are manipulated to retract the core wires 173, thereby separating the traction assembly 170 from the first clip arm 110 and the second clip arm 120 and separating the tightening tube 140 from the metal tube preceding the sheath 171. As a result, the middle clip arm 150 is removed from in between the two separators without being stuck due to interference with the first clip arm 110 and/or the second clip arm 120 caused by direct contact therewith, and the separated clip head remains clamping the tissue defect to deliver a closing or hemostatic effect.

### Embodiment 2

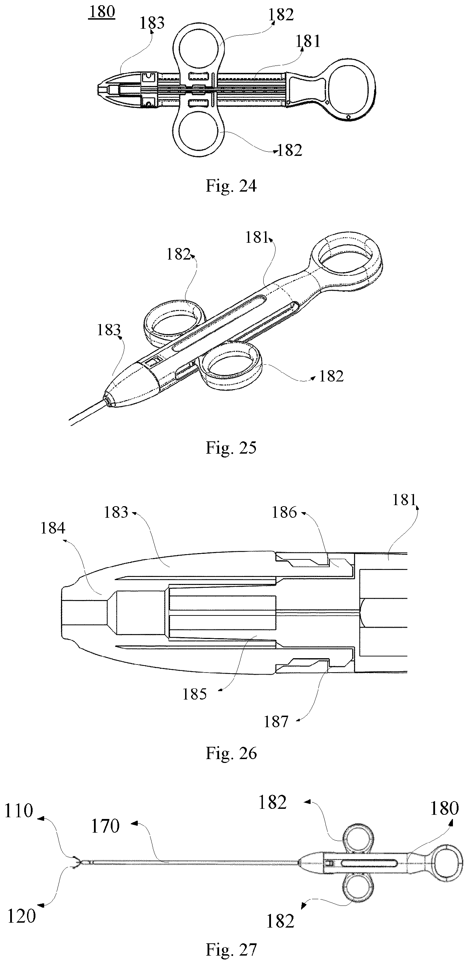

In a second embodiment of the present invention, there is provided a hemostatic clip which differs from that of the first embodiment essentially in including a single control handle 180 capable of controlling both a first clip arm 110 and a second clip arm 120 and a non-branched sheath fixed at a proximal end thereof to the control handle. Specifically, reference is made to Figs. 24, 25 and 26. Fig. 24 is a structural schematic showing the hemostatic clip of the second embodiment. Fig. 25 is an axonometric view of the hemostatic clip of the second embodiment. Fig. 26 is a partially enlarged schematic view of the hemostatic clip of the second embodiment. The control handle 180 includes a handle body 181, two handle pull rings 182 and a limiting nut 183. The two handle pull rings 182 are attached to the handle body 181 so as to be slidable forth and back relative to the handle body 181. The limiting nut 183 is mated to a distal end of the handle body 181.

Reference is now made to Fig. 27. Fig. 27 is a structural schematic showing the hemostatic clip of the second embodiment. In this embodiment, the sheath assembly includes a non-branched sheath, and two core wires in a traction assembly are disposed within a lumen of the sheath. The sheath 171 is proximally fixed to the handle body and the limiting nut 183. The handle body 181 defines a control socket that is open distally, and the limiting nut 183 defines a limiting bore that is open both proximally and distally. The limiting bore is brought at the proximal end thereof into communication with the control socket. The two core wires 173 are passed sequentially through the limiting bore and the control socket and fixed to the respective handle pull rings 182 so that the core wires 173 may be driven to move forth and back by pushing and pulling the respective handle pull rings 182 forth and back relative to the handle body 181. Specifically, referring to Fig. 26, the traction assembly further includes a limiting ring to which the sheath 171 is fixed, and the limiting member 183 defines a ring-shaped limiting member 184 in the limiting bore. The limiting ring is disposed in the limiting bore, and the limiting member 184 is configured to limit distal movement of the limiting ring relative to the limiting nut 183. Moreover, a limiting pin 185 extends in the limiting bore from the distal end of the handle body limits proximal movement of the limiting ring relative to the limiting nut 183. In this way, limited by both the limiting member 184 and the limiting pin 185, the limiting ring can move only in the limiting bore, thus limiting movement of the sheath to which the limiting ring is fixed. The limiting ring can be made of a metal.

As shown in Fig. 26, a limiting finger 186 extends from the proximal end of the limiting nut 183, the handle body 181 is provided with a limiting recess 187 which is snapped with the limiting finger 186, the mating of the limiting nut 183 with the handle body 181 is accomplished by snapped engagement of the limiting finger 186 with the limiting recess 187.

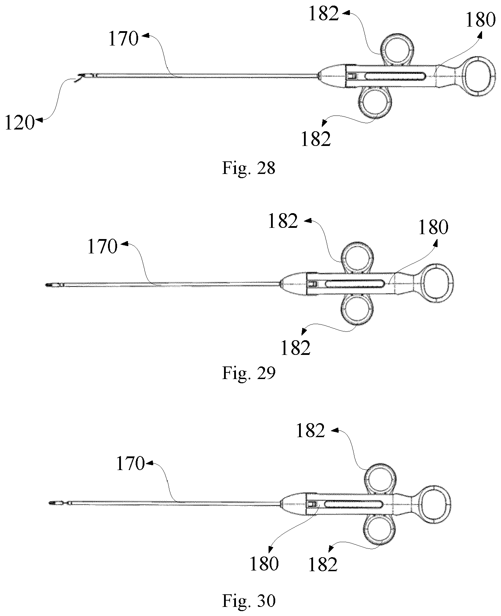

In this embodiment, reference can be made to Figs. 28, 29 and 30 for how the first and second clip arms are opened and closed relative to a middle clip arm under the control of the control handle. Fig. 28 is a structural schematic showing a first clip made up of the first and middle clip arms in a closed configuration. Fig. 29 is a structural schematic showing the first clip made up of the first and middle clip arms and a second clip made up of the second and middle clip arms both in a closed configuration. Fig. 30 is a structural schematic showing a clip head that has been separated from an intermediate assembly, the traction assembly and the control handle.

In the above embodiments, the hemostatic clip has a three-arm structure. The first clip arm 110 and the second clip arm 120 can be independently controlled by individually operating the two control handles 180. This imparts greater flexibility to the device in its surgical applications. Tissue on one side of a lesion is first aligned with the first clip arm 110 and firmly clamped between the first clip arm 110 and the middle clip arm 150, and tissue on the opposite side of the lesion is then aligned with and firmly clamped by the second clip arm 120. This can be accomplished with simple, reliable operations, and the hemostatic clips are suited to the closure of large or complex tissue defects or perforations.

The first clip arm 110 and the second clip arm 120 can be both opened and closed repeatedly without an opening/closing cycle count limit. This allows adjustments in the positional of the device relative to the tissue before it is positioned in a desirable way. Only after this, the clip head may be separated, avoiding an undesirable clamping location, tissue damage or other issues. The middle clip arm 150 serves mainly for anchoring and supporting. It facilitates locating, clamping and retracting tissue around a defect and can be separated and withdrawn with the intermediate assembly 160 when the first clip arm 110 and the second clip arm 120 are both in a closed configuration.

In the above embodiments, the hemostatic clip can be operated by two persons to perform an endoscopic surgical operation involving hemostasis, perforation closure, fistula closure or tissue defect repair, or to be used as a supporting tool for another therapeutic means. As used herein, the term "endoscope" refers to a medical endoscope for therapeutic or diagnostic use, including a gastroscope, a colonoscope, a duodenoscope, an enteroscope, a choledochoscope, a bronchoscope or another endoscope equipped with a working channel (or a working clamp channel). Further, the two handles of the hemostatic clip can be operated by two persons to perform a cavascopic operation or assist a cavascope or another instrument in hemostasis or perforation repair.

As used hereinabove, the terms "proximal" and "distal" describe relative orientations, relative positions and directions between elements or actions, as viewed by an operating physician. Without wishing to be limiting, a "proximal end" usually refers to an end closer to the physician during normal operation, while a "distal end" usually refers to an end that enters the patient first. Additionally, as used hereinabove, the term "or" is generally employed in the sense including "and/or" unless the context clearly dictates otherwise. Further, as used hereinabove, the term "both ends" refers to proximal and distal ends.

The foregoing description presents merely some preferred embodiments of the present invention and is not intended to limit the scope of the present invention in any sense. It is intended that all changes and modifications made by those of ordinary skill in the art in light of the above teachings fall within the scope of the appended claims.

## Claims

1. A hemostatic clip, comprising a first clip arm, a second clip arm, a tightening tube, a middle clip arm, an intermediate assembly, a traction assembly, a control handle and at least two separators,
the control handle, the traction assembly, the intermediate assembly and the tightening tube sequentially connected together along a proximal-to-distal direction, the control handle connected to a proximal end of the traction assembly, a distal end of the traction assembly connected to a proximal end of the intermediate assembly, a distal end of the intermediate assembly connected to the tightening tube,
proximal ends of the first and second clip arms disposed within the tightening tube, the proximal ends of the first and second clip arms connected to the traction assembly, a proximal end of the middle clip arm extending through the tightening tube and fixedly connected to the intermediate assembly, the first and second clip arms disposed on opposing sides of the middle clip arm, the first clip arm making up a first clip together with the middle clip arm, the second clip arm making up a second clip together with the middle clip arm,
the control handle configured to individually cause the first and second clip arms to move forth and back in the tightening tube by means of the traction assembly, thereby causing distal end(s) of the first clip arm and/or the second clip arm to move toward or away from the middle clip arm and thus opening or closing the first clip and/or the second clip, the separators all disposed within a lumen of the tightening tube, at least one of the separators located between the first clip arm and the middle clip arm and at least one of the separators located between the second clip arm and the middle clip arm.

2. The hemostatic clip of claim 1, wherein the tightening tube is integrally formed with all the separators.

3. The hemostatic clip of claim 1, wherein the tightening tube is formed by welding together with all the separators.

4. The hemostatic clip of claim 1, comprising two separators each in the form of a flat sheet.

5. The hemostatic clip of claim 4, wherein the two separators are spaced apart from each other by a distance of from 0.1 mm to 0.8mm.

6. The hemostatic clip of claim 4, wherein the distance between the two separators is 0.2 mm.

7. The hemostatic clip of claim 4, wherein the tightening tube and the two separators are formed from at least two metal sheets by folding and welding.

8. The hemostatic clip of claim 1, wherein the tightening tube is provided therein with limiting slots configured to work with the proximal ends of the first and second clip arms to limit proximal movement of the first and second clip arms relative to the tightening tube.

9. The hemostatic clip of claim 1, wherein limiting protrusions are provided on sides of the first and second clip arms facing away from a tubular wall of the tightening tube and a stop tab is provided on a distal end face of the tightening tube, the stop tab configured to work with the limiting protrusions to limit movement of the first and second clip arms toward a proximal end of the tightening tube.

10. The hemostatic clip of claim 1, wherein the intermediate assembly comprises a sheath-preceding tube, a connecting member and a pull ring attached to the connecting member, a proximal end of the sheath-preceding tube connected to the traction assembly, a distal end of the sheath-preceding tube detachably connected to the tightening tube by the connecting member, the traction assembly configured to cause distal movement of the pull ring, the pull ring configured to separate, as a result of the distal movement thereof, the connecting member from the tightening tube and the sheath-preceding tube, the sheath-preceding tube defining a lumen in which a proximal end of the middle clip arm is accommodated, and the proximal end of the middle clip arm secured to the lumen of the sheath-preceding tube.

11. The hemostatic clip of claim 10, wherein the connecting member is a biased member, which is engaged with the tightening tube and the sheath-preceding tube when in a relaxed configuration and is disengaged from the tightening tube and the sheath-preceding tube when in a compressed configuration.

12. The hemostatic clip of claim 10, wherein the traction assembly comprises a sheath, two core wires, two hooks and two coupling pins, a proximal end of the sheath connected to the control handle, a distal end of the sheath fixedly connected to the intermediate assembly, the two core wires disposed within a lumen of the sheath, a proximal end of each core wire connected to the control handle, a distal end of each core wire fixedly connected to a respective one of the hooks, the two hooks connected respectively to the first and second clip arms by the respective coupling pins, the control handle configured to pull the first and second clip arms forth and back within the tightening tube via the core wires.

13. The hemostatic clip of claim 12, comprising two control handles, wherein the sheath comprises a first proximal sheath branch, a second proximal sheath branch, a distal sheath section and a connecting tube, a proximal end of the connecting tube fixedly connected to distal ends of the first and second proximal sheath branches, a distal end of the connecting tube fixedly connected to a proximal end of the distal sheath section, a distal end of the distal sheath section fixedly connected to the sheath-preceding tube, proximal ends of the first and second proximal sheath branches fixedly connected to the respective control handles, the two core wires passed sequentially through the distal sheath section, the connecting tube and respectively the first and second proximal sheath branches and connected to the respective control handles.

14. An auxiliary system comprising an endoscope and the hemostatic clip of any one of claims 1 to 13,
the endoscope comprising an endoscopic channel in which the hemostatic clip is disposed, the endoscopic channel configured for delivery and traction of the hemostatic clip.
